# EUROPEAN PATENT SPECIFICATION

(11) **EP 0 548 353 B1**
(45) Date of publication and mention of the grant of the patent: **10.04.1996**
(21) Application number: 92916289.9
(22) Date of filing: 09.07.1992
(51) Int. Cl.: A61M 5/14, A61M 5/162

(54) **ENHANCED INJECTION SITE**
Verbesserte Injektionsstelle
SITE D'INJECTION AMELIORE

(30) Priority: 16.07.1991 US 730764
(43) Date of publication of application: 30.06.1993
(73) Proprietor: BAXTER INTERNATIONAL INC., Deerfield, IL 60015 (US)
(72) Inventor: RICHARDSON, James, Schaumburg, IL 60194 (US); LAL, Birendra, K., Lake Zurich, IL 60047 (US)
(74) Representative: MacGregor, Gordon
(86) International application number: US9205744
(87) International publication number: WO9301847

(56) References cited:
- GB-A- 2 033 230
- US-A- 4 133 441

## Description

### TECHNICAL FIELD

This invention relates to an improved septum construction for use in injection sites and closures for a variety of medical applications.

### BACKGROUND OF THE INVENTION

Septum materials of pierceable resilient and resealable compositions have been used far many years in medical applications. For example, such septums are used in unitary, molded injection sites typical in Y- or T-type sites in intravenous administration sets or in blood collection sets. Septums are also utilized in port closures or stoppers for medical containers or flexible fluid bags. One typical structure of these septums includes a needle-receiving surface integral with a central or core portion, a tail portion and a skirt portion, both carried by the core. Prior to insertion into a tubular housing unit, the tail and skirt portions extend away from the core in opposite directions. As inserted into the housing, the tail is retained in the housing and the skirt is folded back to grippingly contact the outer surface of the housing. The tubular housing then carries the core in compression.

Several considerations are given in the construction of such an injection site or closure. Among these considerations, for example, is the need of the health care professional being able to insert and withdraw a standard hypodermic needle through the site or closure with relative ease and security. In addition, the septum itself must remain in position within the site or closure housing despite back pressures which may be created. These forces --- insertion, removal and push-off --- depend upon or relate to the compression achieved by the interface between the housing and the septum.

The prior art addresses some of these considerations, in particular the insertion force, by suggesting the septum utilized should have reduced effective thickness of material through which the needle must be inserted. For example, U.S. Patent No. 2,372,182 provides a container closure with a relatively thin centrally located portion which may be readily penetrated or pierced by a hypodermic needle. U.S. Patent 3,017,050 discloses a closure for a blood sample collection apparatus having a reduced-thickness area created by a central void. The void reduces the effective thickness of rubber through which the needle must be inserted.

Further, when a needle pierces the septum it passes through the core portion in order to access the fluid flow path, for example, of an I.V. tube in communication with an injection site housing. As the needle is inserted, it cuts a path through the core. When the needle is withdrawn, the path is generally resealed within the core portion in a reliable manner. Again, this ability to reseal is related to the compression forces exhibited by the site housing on the septum. However, after repeated insertions of a needle, sufficient numbers of paths have been created so that reliable resealability of the septum is questionable.

For various reasons, the septum material of these injection sites has a limited life or duration. The duration of a septum can be measured in terms of the number of needle insertions which can be made into the septum before leaking of fluid out of the injection site occurs. Because of this limited duration of the septum material, I.V. administration sets are often recommended and typically used for a maximum of 24 to 48 hours. In addition, the medical community has had to cope with some leaking injection sites in I.V. administration sets and stoppers or closures on flexible medical containers.

In accordance with this invention, an improvement to the septum construction has been discovered which has surprisingly resulted in significantly increasing the duration of the septum without adversely effecting the insertion, removal or push-off forces.

US-A-4133441 discloses a resealing, needle-pierceable, resilient septum having the features of the precharacterising part of Claim 1. No reference is made to the axial thickness of the core portion of the septum, but the drawings show a diameter/axial length ratio of about 2/1.

The distinguishing feature of the present invention is set out in the characterising part of Claim 1.

Previously septums were produced with an outer diameter of the core in the approximate range of 1.6 to 1.7 times the core axial thickness. It has been learned that even a 20% increase in the thickness of the core results in a significant increase in the number of needle insertions before the septum is sufficiently "cored", thereby effecting the resealing integrity of the septum. Contrary to the prior art teachings, such an increase in the core thickness surprisingly does not adversely affect insertion, removal and push off forces.

In accordance with the present invention, the ratio of diameter/axial length of the core portion is no greater than 1.4.

The ratio may be no less than 1.0, so that the ratio is in the range 1.0 to 1.4.

The thickness of the core portion may be 3.5 to 4.5 mm (0.14 to 0.18 inch) and the outer diameter of the core portion at least 4.7 mm (0.188 inch). While it is entirely possible to increase the core thickness 100% so that the core and tail portion of the septum are a solid, uniform section, the insertion and removal forces required for needle insertion through such a solid section may become unacceptable to some health care professionals

### BRIEF DESCRIPTION OF THE DRAWINGS

FIG. 1 is a perspective view, partly broken away in vertical section, of a Y-type injection site of an I.V. administration set showing the improved septum design of this invention;
FIG. 2 is a cross sectional view of a prior art molded septum before insertion into a tubular housing;
FIG. 3 is a cross sectional view of the improved septum of this invention prior to insertion into a tubular housing;

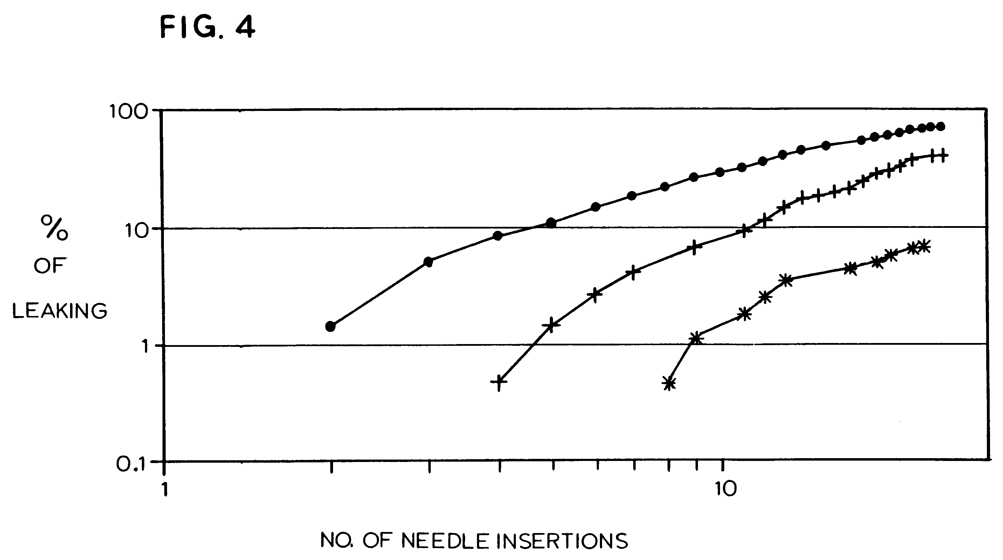

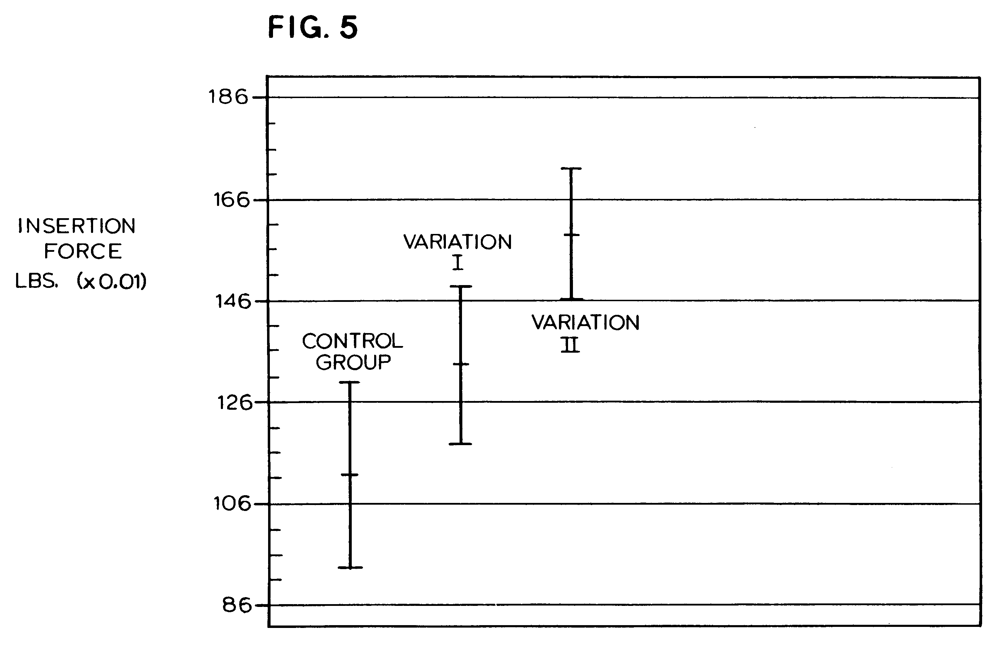

FIGS. 4-7 represent the coring effects of repetitive needle insertion and insertion, removal and push-off forces, and septum to shoulder gap manufacturing tolerances of three separate septum samples housed in Y-type injection sites of the general type described in this application. The control group indicated by "." in FIG. 4 or by "Control Group" in FIGS. 5-7, has septums with cores of a commercially available thickness. The second group of septums indicated by "+" in FIG. 4 or by "Variation I" in FIGS. 5-7, has septums with cores approximately 25% thicker than the Control Group. The third group of septums indicated by "*" in FIG. 4 or by "Variation II" in FIGS. 5-7, has septums with cores approximately 50% thicker than the Control Group.

### DETAILED DESCRIPTION OF THE INVENTION

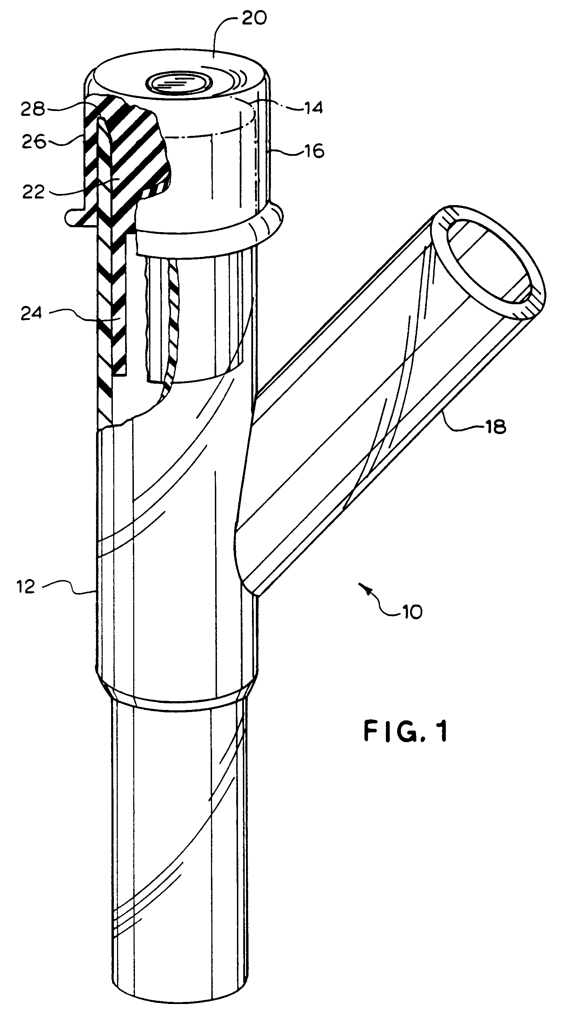

Referring to Figure 1 as an example, a unitary molded Y-type injection site housing 10 typical of an I.V. administration set, has a tubular housing portion 12 having at one end an opening 14 adaptable to receiving a septum 16 of the construction of the subject invention. The second end of housing portion 12 fits integrally with a primary I.V. line, not shown in the Figures, which continues to the distal end of the I.V. administration set. A side branch or arm 18 intercepts housing portion 12 below opening 14. Both housing portion 12 and side arm 18 have internal fluid flow paths which are in communication with the flow path of the primary I.V. line, permitting the flow of fluid through the I.V. administration set.

Preferably made from a latex material, septum 16 is shown to comprise a central, needle-receiving surface 20 integral with a central or core portion 22 and spanning opening 14. The core 22 having a cylindrical shape, carries a tail portion 24 which extends in a direction away from core 22 and a skirt portion 26 extending in an opposite direction from core 22 relative to tail 24.

To install septum 16 into the tubular housing portion 12 of the Y-type injection site, the tail 24 of the septum 16 is inserted through opening 14 and is retained in housing portion 12 by compressive forces created by the interaction between housing portion 12 and both tail portion 24 and core 22. Once fully inserted, core 22 is positioned inside housing portion 12 so that needle-receiving surface 20 spans opening 14. The skirt portion 26 is then folded to grippingly surround the outside of housing portion 12.

The housing portion 12 is designed to maintain septum 16 in a compressed state, thereby assisting in both the retention and resealability of septum 16, even after repeated insertions of a needle. To assist in achieving the necessary compression, the inner diameter of the housing portion 12 and the outer diameter of the core 22 are appropriately sized as one skilled in the art can appreciate, to achieve the compression state sought. Typically, currently available injection site products have a gap 28 of minimal size. The sizing of housing portion 12 and core 22 take into consideration the typical manufacturing tolerances of gap 28 which adversely effects the retention of the compressed state for resealability.

To further assist in the retention of septum 16, shrink bands, not shown in the Figures, are often positioned circumferentially about folded skirt portion 26. In this manner shrink bands reside on the outside of the entire structure to serve as a compression member to the entire assembly.

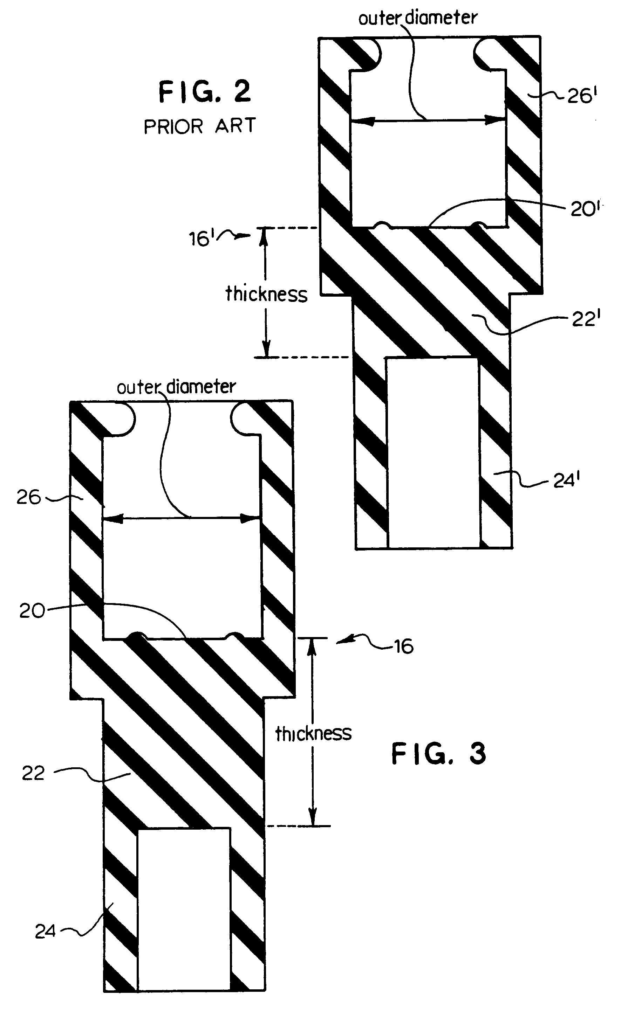

Shown in Figure 2, previous septums 16' were produced with cores 22' having an outer diameter of the core 1.5 times greater than the core thickness. By increasing the thickness of the core by preferably 20-50% without other dimensional changes, significant increases are observed in the number of needle insertions prior to fluid leakage occurrences. Preferably, the increase to the thickness results in a total core thickness ranging from approximately 3.5 to 4.5 mm (0.14 to 0.18 inch) where an outer diameter of the core is approximately 4.5 to 4.8 mm (0.18 to 0.19 inch). In addition, the critical sizing of gap 28 has greater flexibility or higher tolerance, from a manufacturing perspective, since a greater length of the core 22 is in a compressed state.

Figure 4 represents the percent of units which leak after repeated needle insertions, as observed in three different septum groups housed in Y-type injection site housings of the general type described in this application. Repetitive insertion of a needle into a septum is known to cause physical damage to the septum. This damage, known in the medical community as coring or laceration, can result in subsequent leakage through the septum. The data represented in Figure 4 relates the number of needle insertions possible before each of the different septum groups is sufficiently cored so that resealing integrity is effected. From the Weibull plot of this data, a decrease in leaking units is observed as the core of the septum tested is increased in thickness, thereby indicating a longer duration of the septum with the increase thickness in the core.

Figure 5 represents the same three septum groups with regard to the insertion force, as measured in pounds, required to insert a standard 18 gauge hypodermic needle into each of the septum groups. The data of this Figure represents the mean insertion force with a 90% confidence level. Despite the significant increases to the septum thickness, the increase in the insertion force remains minimal.

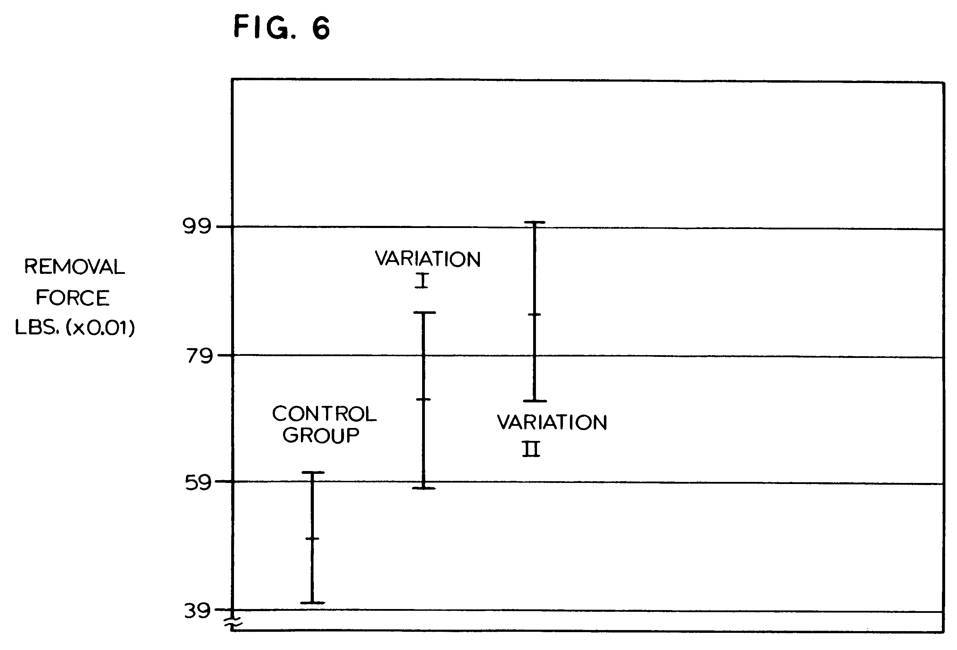

Figure 6 represents data obtained on the same three septum groups with regard to the removal force, as measured in pounds, required to remove a standard 18 gauge hypodermic needle from each of the septum groups. The data of this Figure represents the mean removal force with a 90% confidence level. Similar to the results for insertion force, a significant increase in the septum thickness resulted in a minimal increase in removal force.

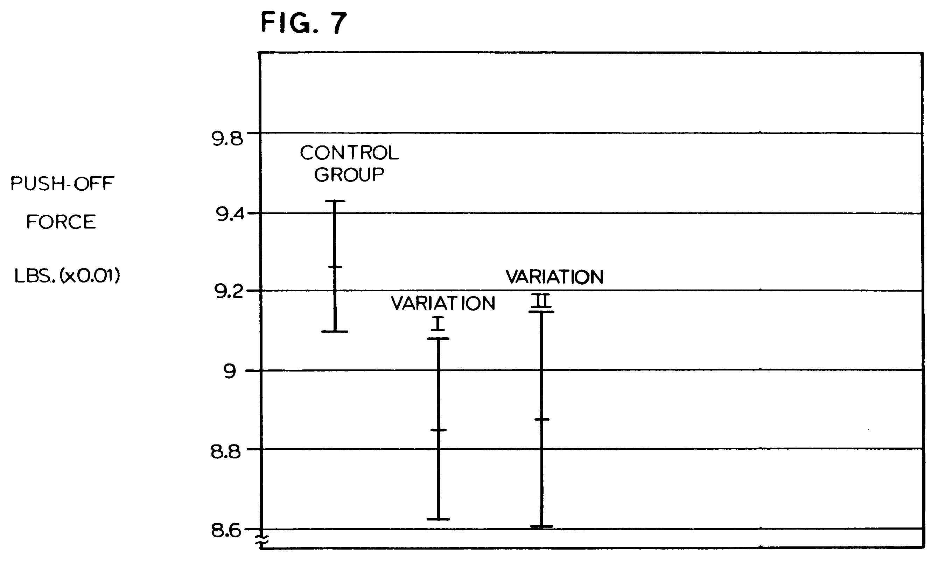

Figure 7 represents the push-off force, with 90% confidence levels, which is required to physically remove the three different septums from the Y-type injection site housing of the general type described in this application. Measured in pounds, the data indicates that minimal reduction in push-off force is observed with an increase septum thickness.

Concluding from this functional data, one skilled in the art can appreciate that the minimal performance differences between septum groups II and III indicates that a greater variation in gap sizing can be tolerated in the manufacturing environment.

Numerous modifications come to mind, for example, the housing portion receiving the septum need not be strictly tubular in shape provided that the septum is held by compression with an interface of the housing portion.

## Claims

1. A resealing, needle-pierceable, resilient septum comprising a cylindrical core portion (22) for compressive retention in a tubular port and having a needle-receiving surface (20) at one end, an annular skirt portion (26) carried by the core portion and extending axially of the needle-receiving surface (20), the skirt portion being outwardly foldable over the end of a tubular port, in which the core portion is retained, to grip the outer surface of the port, and an annular tail portion (24) carried by the core portion (22) and extending from the core portion in the opposite direction to the skirt portion (26) for compressive retention in the port,
characterised in that the outer diameter of the core portion (22) is no greater than 1.4 times its axial thickness.

2. The septum of Claim 1 wherein the outer diameter of the core portion is no less than about 1.0 times its axial thickness.

3. The septum of Claim 1 or 2 wherein the thickness of the core portion is about 3.5 mm (0.14 inch) and the outer diameter of the core portion is about 4.5 mm (0.18 inch).

4. The injection site of Claim 3 wherein the thickness of the core portion is at least 3.5 to 4.5 mm (0.14 to 0.18 inch), and the outer diameter of the core portion is at least 4.7 mm (0.188) inch.

5. An injection site comprising a resealing, needle-pierceable, resilient septum (16) according to any preceding claim and a unitary, molded housing (10) including a generally tubular portion (12) with outer and inner surfaces and an opening (14) at one end, the septum (16) being received and retained under compression in the generally tubular portion (12) with the skirt portion (26) folded over the outer surface of the portion (16) in gripping contact therewith.

6. An injection site according to Claim 5, including a branch arm (18) which intercepts the generally tubular housing portion (12) in fluid flow communication therewith.

## Patentansprüche

1. Wiederverschließendes, von einer Nadel durchstechbares, elastisches Septum, das folgendes aufweist: einen zylindrischen Kernbereich (22) zur Festlegung unter Kompression in einer rohrförmigen Öffnung, der eine Nadelaufnahmefläche (20) an einem Ende hat, einen ringförmigen Mantelbereich (26), der von dem Kernbereich getragen wird und in Axialrichtung der Nadelaufnahmefläche (20) verläuft, wobei der Mantelbereich über das Ende einer rohrförmigen Öffnung, in der der Kernbereich festgelegt ist, nach außen umfaltbar ist, um die äußere Oberfläche der Öffnung zu umgreifen, und einen ringförmigen Endbereich (24), der von dem Kernbereich (22) getragen ist und von dem Kernbereich in der zu dem Mantelbereich (26) entgegengesetzten Richtung verläuft, um in der Öffnung unter Kompression festgelegt zu sein, dadurch gekennzeichnet, daß der Außendurchmesser des Kernbereichs (22) nicht größer als das 1,4fache seiner axialen Dicke ist.

2. Septum nach Anspruch 1, wobei der Außendurchmesser des Kernbereichs nicht kleiner als ca. das 1,0fache seiner axialen Dicke ist.

3. Septum nach Anspruch 1 oder 2, wobei die Dicke des Kernbereichs ca. 3,5 mm (0,14 inch) und der Außendurchmesser des Kernbereichs ca. 4,5 mm (0,18 inch) ist.

4. Septum nach Anspruch 3, wobei die Dicke des Kernbereichs wenigstens 3,5 bis 4,5 mm (0,14 bis 0,18 inch) und der Außendurchmesser des Kernbereichs wenigstens 4,7 mm (0,188 inch) ist.

5. Injektionsstelle, die ein wiederverschließendes, von einer Nadel durchstechbares, elastisches Septum (16) nach einem der vorhergehenden Ansprüche und ein einstückiges Formgehäuse (10) aufweist, das einen allgemein rohrförmigen Bereich (12) mit einer äußeren und einer inneren Oberfläche und einer Öffnung (14) an einem Ende umfaßt, wobei das Septum (16) in dem allgemein rohrförmigen Bereich (12) aufgenommen und unter Kompression festgelegt ist, wobei der Mantelbereich (26) über die äußere Oberfläche des Bereichs (12) in umgreifender Berührung damit umgefaltet ist.

6. Injektionsstelle nach Anspruch 5, die einen Zweigarm (18) aufweist, der den allgemein rohrförmigen Gehäusebereich (12) in Fluiddurchflußverbindung damit schneidet.

## Revendications

1. Obturateur élastique, perçable par une aiguille et étanche, comprenant une partie centrale cylindrique (22) de rétention compressive dans un passage tubulaire et présentant une surface (20) de réception d'aiguille à une extrémité, une partie en forme de jupe annulaire (26) portée par la partie centrale et s'étendant axialement de la surface (20) de réception d'aiguille, la partie de jupe étant repliable extérieurement sur l'extrémité du passage tubulaire dans lequel la partie centrale est retenue pour serrer la surface externe du passage, et une partie de queue annulaire (24) portée par la partie centrale (22) et s'étendant de la partie centrale dans la direction opposée à la partie de jupe pour une rétention compressive dans le passage, caractérisé en ce que le diamètre externe de la partie centrale (22) est au plus 1,4 fois son épaisseur axiale.

2. Obturateur selon la revendication 1, dans lequel le diamètre externe de la partie centrale n'est pas inférieur à environ 1 fois son épaisseur axiale.

3. Obturateur selon les revendications 1 ou 2, dans lequel l'épaisseur de la partie centrale est environ égale à 3,5 mm (0,14 inch) et le diamètre externe de la partie centrale est d'environ 4,5 mm (0,18 inch).

4. Obturateur selon la revendication 3, dans lequel l'épaisseur de la partie centrale est au moins comprise entre 3,5 et 4,5 mm (0,14 à 0,18 inch) et le diamètre externe de la partie centrale est au moins de 4,7 mm (0,188 inch).

5. Site d'injection comprenant un obturateur souple, perçable par une aiguille et étanche selon l'une quelconque des revendications précédentes, un corps moulé d'une seule pièce (10) comportant une partie généralement tubulaire (12) présentant des surfaces externe et interne et une ouverture (14) à une extrémité, l'obturateur (16) étant disposé et maintenu en contact serré sur la surface externe de la partie (16).

6. Site d' injection selon la revendication 5, comprenant un bras latéral (18) qui intercepte et qui est en communication fluidique avec la partie de corps (12) sensiblement tubulaire.
